# EUROPEAN PATENT APPLICATION

(11) **EP 2 044 852 A1**
(43) Date of publication of application: **08.04.2009**
(21) Application number: 07117737.2
(22) Date of filing: 02.10.2007
(51) Int. Cl.: A23L 1/30, A61K 36/27

(54) **Compositions with colloidal particles of steroidal glycosides**

(71) Applicant: Unilever N.V., 3013 AL Rotterdam (NL)
(72) Inventor: Velikov, Krassimir Petkov, 3133 AT Vlaardingen (NL); Abrahamse, Salomon Leendert, 3133 AT Vlaardingen (NL)
(74) Representative: Wurfbain, Gilles L.

(57) **Abstract**

The present invention provides a composition comprising colloidal particles steroidal glycoside, preferably prepared from concentrated Hoodia extracts. Also provided is additive comprising steroidal glycoside.

## Description

### TECHNICAL FIELD

The present invention relates to compositions comprising steroidal glycosides in the form of colloidal particles.

### BACKGROUND OF THE INVENTION

Extracts obtainable from plants of the *Asclepiadaceae* family, particularly the *Hoodia* genus (formerly the *Hoodia* and *Trichocaulon* genera) have been shown to have an appetite suppressant activity and are useful in weight management products. US Patent 6,376,657 discloses that these plants contain steroidal glycosides. US Patent 6,376,657 also discloses processes to extract steroidal glycosides from *Hoodia* plants.

Unfortunately, it is difficult to formulate suitable products comprising such steroidal glycosides or extracts, especially of the concentrated extracts (i.e., those comprising more than 60% steroidal glycosides) and pure compounds, because the concentrtated extracts and the pure compounds are solid at room temperature. Moreover, they are not soluble in water and poorly soluble in edible fats. Melting followed by emulsification is difficult and commercially unattractive, as their melting temperature is also very high (> 200°C).

As a consequence, these materials are very difficult to disperse in a product. The poor solubility of steroidal glycosides in oil or water leads to the formation of large solid aggregates, which impart a gritty (sandy) texture poor stability of the foods. In addition, such apprioach leads to large particles that sediment and/or stick to the container/package walls. Furthermore, the poor dispersability of the material makes cleaning of the food processing equipment difficult and can lead to problems when the material is mixed with or dispersed in other food ingredients. Finally, even when acceptable food products can be produced, the bioavailability of the active compounds is not optimal due to the slow digestion of large and very hard particles.

Accordingly, there is a need for a a stable and organoleptically acceptable aqueous dispersion of steroidal glycosides. A further object of the invention is to provide a process for preparation of such formulaitons. A further object of the invention is to provide food, personal care or pharmaceutical products comprising the colloidal particlesd/or the dispersions.

The present invention is based at least in part on the discovery that colloidal particles of defined small particle size can be prepared from Hoodia plant extracts concentrated in steroidal glycosides, i.e. more than 60% steroidal glycosides.

### SUMMARY OF THE INVENTION

In a first aspect, the present invention relates to a composition comprising colloidal particles of *Hoodia* plant extract comprising more than 60% of steroidal glycosides.

In a second aspect, the present invention relates to a process for producing colloidal particles comprising steroidal glycosides.

In a third aspect, the present invention provides a method of manufacturing a composition comprising colloidal particles comprising steroidal glycosides, the process comprising the steps of:
i) preparing colloidal particles from Hoodia extract comprising more than 60% steroidal glycosides;
ii) recovering the colloidal particles; and
iii) combining the colloidal particles with a supporting medium.
   Such a process allows for manipulation of the properties of the colloidal particles independently of the supporting medium.

In a forth aspect, the present invention provides a method of manufacturing a composition by in situ generation of colloidal particles comprising steroidal glycosides.

Another aspect of this current invention is food, personal care and pharmaceutical products comprising steroidal glycosides in the form of colloidal particles.

### DETAILED DESCRIPTION OF THE INVENTION

Except in the examples, or where otherwise explicitly indicated, all numbers in this description indicating amounts of material or conditions of reaction, physical properties of materials and/or use are to be understood as modified by the word "about." All amounts are by weight, unless otherwise specified.

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

As used herein, the term "particle size" refers to the maximum length of a particle in any dimension. Particles may be spherical, non-spherical or a mixture thereof. Thus, if a particle is spherical then the particle size refers to the diameter of the particle.

### Dynamic Light Scattering (DLS)

DLS measurements were performed using a Zetasizer Nano ZS instrument (Malvern Instruments, Malvern, UK). This instrument records intensity fluctuations of scattered light at a fixed angle from clear samples containing particles. These fluctuations are converted into an autocorrelation function spanning time scales between picoseconds and seconds. The instrument software fits intensity- and volume-weighed size distributions to the recorded autocorrelations. Samples were measured without any dilution at 25°C. The viscosity of the samples was assumed to be that of water in all cases and a refractive index of 1.59 was used in the analysis. Average particle size is quoted as volume - averaged mean particle size. Where more than one peak was present in the data, the particle size corresponding to the maximum of each peak is given.

### Transmission electron microscopy (TEM)

Electron microscopy imaging on the colloidal particles was performed using Transmission Electron Microscopy (TEM). To prepare samples, a few droplets of the colloidal suspension were transferred to a carbon coated electron microscopy grid by dipping the grid into the suspension. The excess fluid was drained and the grid was left to air-dry. The grid was examined by Tecnai Sphera 200 kV transmission electron microscope operating at 200 kV (FEI Company, Hillsboro, OR, USA). The images were digitally recorded on a Tietz 214 cooled CCD camera (TVIPS GmbH, Gauting, Germany).

"Steroidal glycoside" as used herein means a steroidal (four fused rings in a 6-6-6-5 pattern), further comprising at least one side group substitution which is a glycoside (a molecule in which a sugar group is bonded through its anomeric carbon to another group via an O-glycosidic bond), preferably a deoxy or dideoxy glycoside, and another side group comprising a carbonyl (-C=O) group, preferably located on the 5-membered ring and most preferably further comprising a tigloyl group 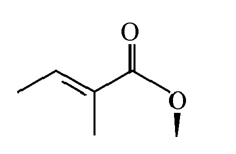 on carbon number 12.

All amounts, parts, ratios and percentages used herein are by weight, unless otherwise specified.

### THE COMPOSITION

The colloidal particles can consist of pure *Hoodia* extract, which contains at least 60% steroidal glycosides, or from mixtures of such extract and other hydrophobic substances that can be processed together with the extract in form colloidal particles in the preferred embodiment of this invention. Examples of such materials can be fats, waxes, functional actives (e.g. the group oil soluble vitamins), vitamins, nutraceuticals, drugs, minerals, water insoluble polymer polymers and biopolymers like zein, cellulose).

In particular, the colloidal particles contain at least 2% steroidal glycoside by weight of the particles, preferably at least 5%, more preferably at least 10%, most preferably at least 40%, more preferably at least 60%, more preferably at least 80% and most preferably at least 99.9% by weight of the composition. The composition comprises from 0.01 to 90% of the particles, preferably 0.05 to 80, more preferably 0.1 to 70, most preferably 0.1 to 60%.
Of particular advantage are colloidal particles comprising more than 60% steroidal glycosides as these can now be prepared according to the invention, in a commercially acceptable manner, directly from *Hoodia* extract comprising more than 60% steroidal glycosides.

The steroidal glycoside is present in the composition in the form of colloidal particles. At least 90% by weight of the particles, preferably at least 95%, have a particle size from 10,000 nm to 10 nm, more preferably from 5000 nm to 50 nm, most preferably in order to obtain optimum stability from 1000 nm to 100 nm.

Typically the particles will be dispersed in a supporting medium. Preferably the supporting medium will make up the bulk of the composition and determine, to a large extent, its sensory and physical characteristics. Thus it is preferred that the composition comprises the steroidal glycoside in an amount of less than 50% by weight of the composition, more preferably less than 10% and most preferably from 5% to 0.05%. The supporting medium may be any suitable substance and will depend to a large extent on the intended end use of the composition. Typically, however, the supporting medium will be a liquid, dispersion (single or duplex emulsion, foam or suspension), gel, solid, or a mixture thereof. The supporting medium may be aqueous (comprise at least 50% water by weight of the supporting medium) or non-aqueous. In the most preferred embodiment, the properties of the particles, especially their size and surface properties, and those of the supporting medium, especially its viscosity and polarity, are selected such that the particles form a stable colloidal dispersion in the supporting medium. Stable dispersion is defined here as a system where particles do not undergo any aggregation (i.e. do not change size in time). The dispersion is preferably stable such that no visible sedimentation of the particles occurs over a period of at least 3 days at a storage temperature of 20°C, more preferably over a period of at least 1 month and most preferably at least 6 months.
In a particularly advantageous embodiment of the invention, the steroidal glycoside is selected from the group consisting of appetite suppressing steroidal glycosides that can be extracted from plants of the genus *Trichocaulon* or of the genus *Hoodia,* derivatives, salts and analogues of said steroidal glycosides and mixtures thereof. According to another equally preferred embodiment, the plant extract is an extract from plants of the group comprising the genus *Trichocaulon* and the genus *Hoodia,* said extract having appetite suppressant activity. More preferably, said plant extract is selected from the group consisting of *Trichocaulon piliferum* extracts, *Trichocaulon officinale* extracts, *Hoodia currorii* extracts, appetite suppressant *Hoodia gordonii* extracts, *Hoodia lugardii* extracts and mixtures thereof. US Patent 6,376,657, incorporated by reference herein, describes the preparation of a suitable extract comprising steroidal glycosides from the genus *Hoodia,* said steroidal glycoside having appetite suppressant activity. *Hoodia gordonii* extract is especially preferred.

"Extract" as used herein includes solvent-extracted liquid, solid or spray-dried or freeze-dried forms of extracts, sap, which also may be purified, partially purified, concentrated and/or fractionated, or otherwise concentrated plant preparation. Solvent extracted forms of the extract are preferred due to higher purity and process efficacy.

According to an especially preferred embodiment, the steroidal glycoside has the general structural formula (I) or a salt or ester thereof: 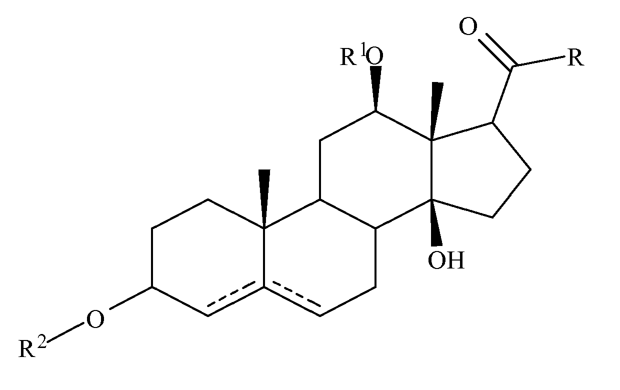 wherein
R = alkyl;
R1 = H, alkyl, tiglyol, benzoyl or any other organic ester group;
R2 = H or one or more 6-deoxy carbohydrates, or one or more 2,6-dideoxy carbohydrates, or glucose molecules, or combinations thereof; and wherein the broken lines indicate the optional presence of a further bond between carbon atoms C4 and C5 or between carbon atoms C5 and C6.

Particularly preferred steroidal glycosides are analogs of Compound of Formula 1, including Compounds of Formula (2) through Formula (8), and mixtures thereof, since these are obtainable from the preferred *Hoodia* plants.

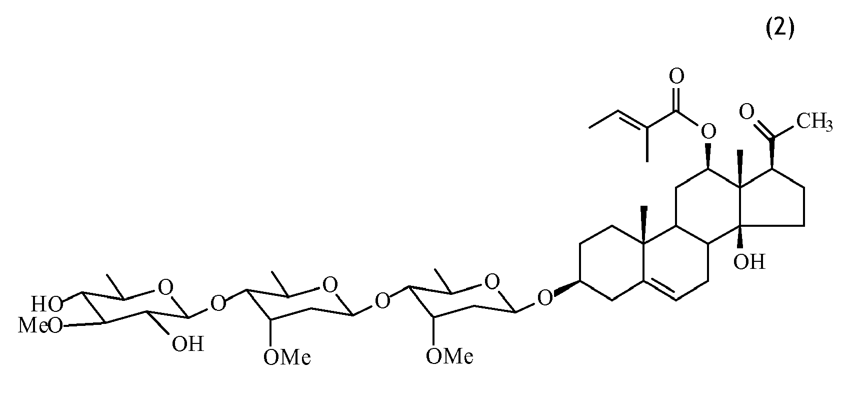

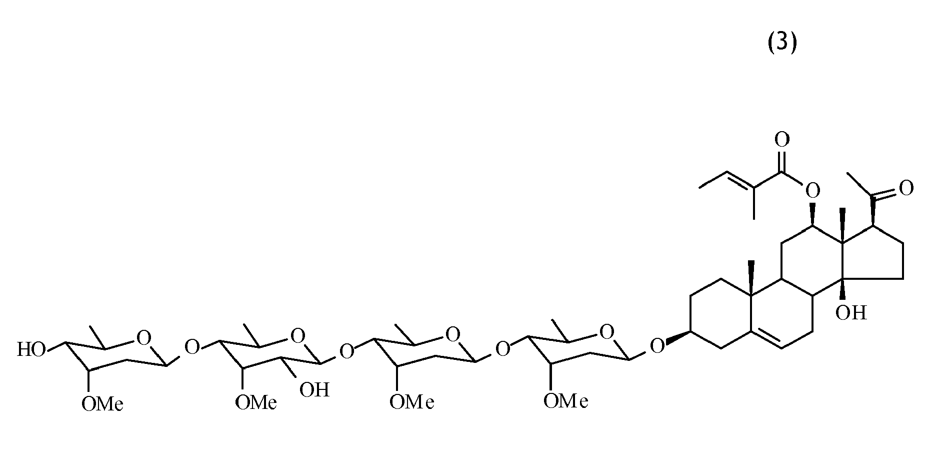

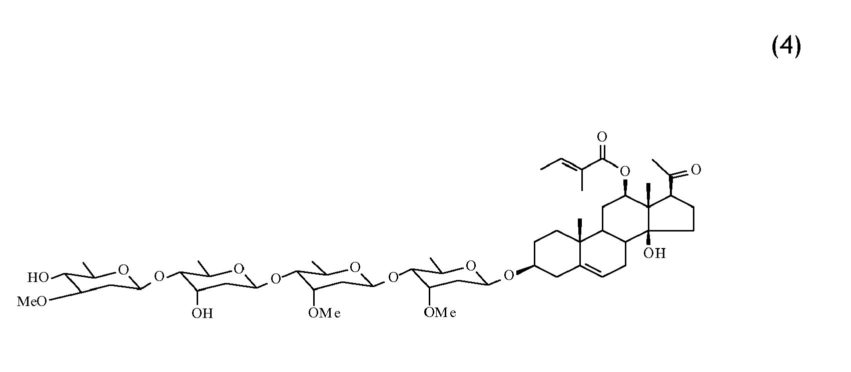

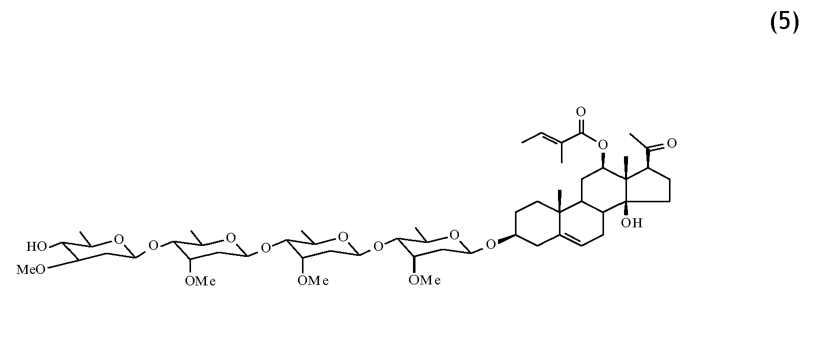

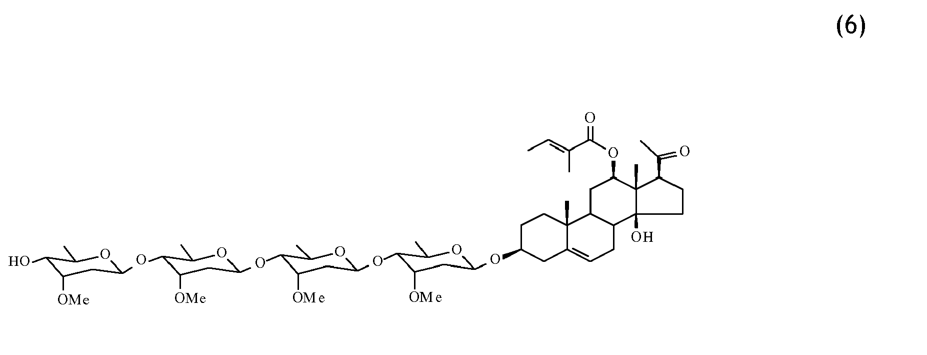

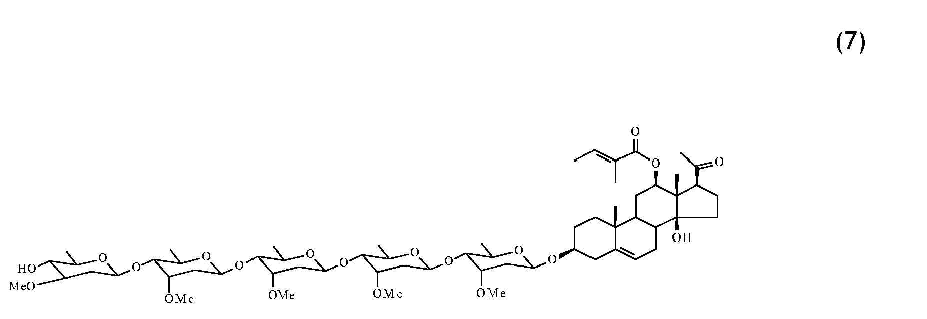

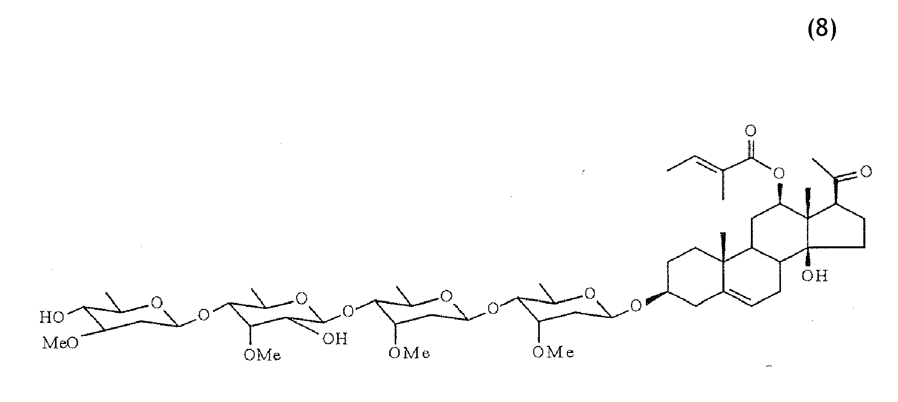

Other steroidal glycosides not specifically mentioned herein may be included in the inventive product. It will be understood that the invention also encompasses isomers, derivatives, salts, esters and analogs of the steroidal glycosides (preferably, biologically active) and mixtures thereof.

Steroidal glycoside concentrations are determined using high performance liquid chromatography (HPLC) with UV detection after extraction or dissolution.

Approximately 20 mg of the material is dissolved in 50 ml of methanol by sonication for 10 minutes. After filtration 1 ml of the filtrate is evaporated to dryness and reconstituted in 1 ml acetonitrile/water (50/50 v/v).

Steroidal glycosides are measured by LC-UV at 220 nm. To this end 20 µl of the extracts are injected onto a Zorbax RX-C8 analytical column of 250 x 4.6 mm packed with 5 µm particles and equipped with a Zorbax RX-C8 guard column of 12.5 x 4.6 mm packed with the same stationary phase. The column system is held at 40°C. Gradient elution is performed starting at 41.2% acetonitrile/methanol (85/15 v/v) and 58.8% water/methanol (85/15 v/v) at a flow rate of 1 ml/min. Initial conditions are held for 10 minutes before being linearly increased to 88.2% acetonitrile/methanol (85/15 v/v) and 11.8% water/methanol (85/15 v/v) over 30 minutes. After a final hold of 5 minutes the system is re-equilibrated to the starting conditions.

Compound of Formula 2 of any known purity (95% was used in this case) is used for calibration. Compound 2 may be isolated from an extract of dried *Hoodia* gordonii using preparative liquid chromatography or may be synthesized (see e.g. US Patent 6,376,657, incorporated by reference herein). A stock solution at 100 µg/ml is prepared in acetonitrile/water (1/1 v/v) and further dilutions are prepared to yield additional calibration standards at 75, 50, 20, 10 and 5 µg/ml. UV response at 220 nm is used for quantification against the Compound 2 calibration line. Relative response factors based on molecular weight are used to quantify the steroidal glycosides against the Compound 2 calibration line. Steroidal glycosides are defined as all peaks eluting after 15 min that are not present in the blank acetonitrile/water (1/1 v/v) sample. For the compounds of Formula 2 - 8 the specific relative retention times and response factors, are summarized in the table below.

Relative retention times and response factors of some steroidal glycosides

| Compound | Relative retention time vs. Compound 2 | Response factor vs. Compound 2 |
|---|---|---|
| formula 2 | 1.000 | 1.000 |
| formula 8 | 1.066 | 1.164 |
| formula 3 | 1.128 | 1.164 |
| formula 4 | 1.191 | 1.130 |
| formula 5 | 1.292 | 1.146 |
| formula 6 | 1.328 | 1.146 |
| formula 7 | 1.399 | 1.309 |

The other steroidal glycosides peaks eluting after 15 minutes have a response factor of 1.081 vs. Compound 2.

The present invention is particularly useful for processing extracts comprising more than 60% steroidal glycosides, even more preferably more than 70%, most preferably more than 80%, and optimally more than 90%.

### Pharmaceutical and Cosmetic Compositions

The pharmaceutical and cosmetic compositions of the present invention may be suitable for any form of administration including oral, topical and/or intravenous administration. The form of the composition may, among others, be a tablet, pill, lozenge, paste, lotion, gel, cream, liquid (including emulsion and/or suspension), spray (including aerosol spray), foam or powder.

The pharmaceutical or cosmetic composition comprises a pharmaceutically or cosmetically acceptable vehicle which may act as a diluent, dispersant or carrier for the complex in the composition. The vehicle may be aqueous or anhydrous.

Water, when present, will be in amounts which may range from 5 to 99%, more preferably from 20 to 70%, optimally between 40 and 70% by weight of the composition.

Emollient materials may also be included in the vehicle. These may be in the form of silicone oils and/or synthetic esters.

Thickeners may also be utilized as part of the vehicle, as may sugars, plasticizers, antioxidants, antimicrobial agents, chelating agents, buffers, coloring agents, pigments, opacifiers, surfactants, propellants, flavours and/or perfumes.
Preferred cosmetic compositions are those suitable for application to human skin and preferably include a skin benefit agent in addition to the complex. Suitable skin benefit agents include anti-aging, anti-inflammatory, wrinkle-reducing, skin whitening, anti-acne, sunscreen (including UV-absorbing) and/or sebum reduction agents. Examples of these include alpha-hydroxy acids, beta-hydroxy acids, polyhydroxy acids, hydroquinone, t-butyl hydroquinone, vitamin B and C and their derivatives, micronised metal oxides, retinoids, betulinic acid, vanillic acid, allantoin, a placenta extract, hydrolactin, resorcinol derivatives, polyphenols, and mixtures thereof.

### Food Compositions

A particularly preferred aspect of the present the invention is the edible aqueous dispersion obtainable by the process according to the invention, comprising one or more steroidal glycosides. The food composition may be, for example, margarine, spread, a confectionery product (such as chocolate or cereal bar), a snack bar, ice cream, dressing, mayonnaise, sauce, bakery product, shortening or cheese. However, it is especially preferred that the food composition is a beverage or a bar.

The food may be dried and contain less than 40% water by weight of the composition, preferably less than 25%, more preferably from 1 to 15%. Alternatively, the food may be substantially aqueous and contain at least 40% water by weight of the composition, preferably at least 50%, more preferably from 65 to 99.9%.

The food preferably comprises nutrients including carbohydrate (including sugars and/or starches), protein, fat, vitamins, minerals, phytonutrients (including terpenes, phenolic and polyphenolic compounds, organosulfides or a mixture thereof) or mixtures thereof. The food may be low calorie (e.g. have an energy content of less than 100 kCal per 100 g of the composition) or may have a high calorie content (e.g. have an energy content of more than 100 kCal per 100 g of the composition, preferably between 150 and 1000 kCal).

The food may also contain sugar, salt, flavours, colours, preservatives, antioxidants, non-nutritive sweetener or a mixture thereof.

The pH of the beverage may, for example, be from 2.5 to 8, preferably 3 to 6, more preferably from 3.5 to 5.

When the food composition is a beverage, it is preferably a protein-based, coffee-based beverage, a tea-based beverage and/or a cocoa-based beverage. Most preferably the beverage is tea-based or protein-based.

The obtained edible aqueous dispersion can be used in can be used in the management of body weight or in the dietary control of obesity.

The preferred product format of the edible appetite suppressant product is a unit serving drink or bar. A bar weight is from 20 to 100 g, preferably from 25 to 75 g, most preferably from 25 to 60 g. A drink has a volume of 80 to 600 ml, preferably 90 to 400 ml, most preferably from 100 to 350 ml. Preferably each unit serving is separately packaged and includes instructions, in particular recommendation of the number of the particular unit serving to be consumed per day.Unit serving products of the invention contain from 50 to 2000mg steroidal glycosides, preferably from 70 to 1500, more preferably from 80 to 1200mg, and optimally from 100 to 1000mg.

The especially preferred unit serving drink according to the invention has the following composition, in addition to the steroidal glycoside:
75-95 wt% (preferably 80-90 wt%) moisture
0.5-10 wt% (preferably 1-7 wt%) protein
0.5-6 wt% (preferably 0.6-5 wt%) fat (including the oils of the inventive oil composition)
3-20 wt% (preferably 4-15 wt%) carbohydrate and
up to 8 wt% (preferably 1-6 wt%) minor components

The especially preferred unit serving bar according to the invention has the following composition, in addition to the steroidal glycoside:
3-30 wt% (preferably 5-25 wt%) moisture
3-30 wt% (preferably 5-25 wt%) protein
3-30 wt% (preferably 5-25 wt%) fat (including the oils of the inventive oil composition)
35-80 wt% (preferably 40-75 wt%) carbohydrate and up to 12 wt% (preferably 1-10 wt%) minor components

The inventive product is used for suppressing appetite and/or controlling obesity in humans. Generally, at least one inventive product should be ingested per day, typically from 1 to 5 per day (per 24 hours), until reaching the desired weight, and then continuing with this regime to maintain the desired weight. Most preferably, from 1 to 3 products are consumed per day for optimum effect. Most preferably, the inventive products are consumed for at least 14 consecutive days.

### THE METHOD

The colloidal particles comprising steroidal glycosides according to the invention may be manufactured from *Hoodia* extract comprising more than 60% steroidal glycosides. Suitable methods include mechanical milling techniques like dry or wet milling processes, or by precipitation or condensation of the extract dissolved in solvents with subsequent separation of the solvent. Suitable solvent based methods are emulsification evaporation methods, emulsification diffusion methods, and solvent-displacement methods.

The undesirable solvent can be removed by evaporation or complete drying of the suspension.

In both variants stabilizer and additives such as emulsifiers, surfactants and (bio) polymers can be used as colloidal stabilizers or also form the colloidal particles phase itself.

Compositions according to the invention may be manufactured in any suitable manner. However in a preferred embodiment the colloidal particles are manufactured independently of the supporting medium. This method comprises the steps of:
i) preparing colloidal particles from *Hoodia* extract comprising more than 60% steroidal glycosides, using a suitable method;
ii) recovering the particles; and
iii) combining the particles with a supporting medium.

The mixing in step (i) is preferably performed in a liquid medium, more preferably an aqueous liquid medium. The mixing is preferably performed under continuous stirring.

The recovery step (ii) usually involves separating the colloidal particles from a liquid medium, for example by drying, sedimentation, filtration or a mixture thereof. Drying may involve spray drying, freeze drying or a mixture thereof. In a most preferred embodiment the complex is recovered in step (ii) as particles, for example in the form of a powder or concentrated suspension. Step (ii) may additionally or alternatively include stabilization of the complex using surface active agents (surfactants, emulsifiers see e.g. Industrial Surfactants (2nd Edition) By: Flick, Ernest W. 1993 William Andrew Publishing/Noyes), polymers, sugars and/or protective colloids.

Examples of suitable stabilizers include but are not limited to polysorbates (e.g. Tween 20-80), sugar ester, gum Arabic, OSA starch, PVP.

The weight ratio (w/w) of the steroidal glycosides to the stabiliser in the aqueous dispersion is less than 10:1. Preferably it is from 10:1 to 1:1,000, more preferably from 100:1 1 to 1:100.

The colloidal particles used in the present invention can be stabilised by means of a biopolymer and their derivatives, such as, polyamides (e.g. proteins and poly(amino acids)), polysaccharides (e.g. cellulose, starch and xanthan), organic polyoxoesters synthesized by bacteria and eukaryotic organisms (e.g. poly(hydroxyalkanoic acids), poly(malic acid), polylactides, polyglycolide, polyanhydrides, polyesteramides and cutin), polythioesters, polyphosphate, polyisoprenoids (e.g. natural rubber or Gutta Percha), polyphenols (e.g. lignin or humic acids), and nucleic acids such as ribonucleic acids and deoxyribonucleic acids. The most preferred biopolymers are polyamides (protein and poly amino acids)) and polysaccharides.

The polyamide (protein) source may be any specific type of protein, e.g. animal (collagens and gelatines), in particular dairy protein, or plant protein. The plan protein sources are for example soy, pea, amaranth, canola (rape), carob, corn, oat, potato, sesame, rice, wheat, lupin protein, or mixtures thereof. These proteins can be intact or partially hydrolysed, and can be used separately or in combination with each other. The preferred protein source is whey protein or soy protein.
The polysaccharide source can be used as stabilisers, particularly polysaccharide gums. Preferred stabilisers are selected from the group of locust bean gum, tamarind seed polysaccharide, alginates, alternan, cellulose, hydroxypropylmethylcellulose (anionic), cell wall polysaccharides from fungi, chitin, chitosan, curdlan, dextran, elsinan, emulsan, gellan, glycogen, glycopeptides, seed gums, hyaluronan, inulin, levan, lipopolysaccharides and other extracellular polysaccharides, peptidoglycans from archaea and bacteria, pectin, pullulan, schizophyllan, scleroglucan, succinoglycan, starch, teichoic acids, teichuronic acids and xanthan gum, guar gum, tara gum, gum arabic, kalaya gum, carrageenan, agar soybean polysaccharides and mixtures thereof. The preferred polysaccharide source is gum arabic.

One or more auxiliary non-polysacccharide stabilisers may be used in addition to the polysaccharide stabiliser(s). In particular, examples of auxiliary stabilisers are glycol alginate esters, methoxy pectin (HM-pectin), sodium carboxymethylcellulose (CMC-Na), propylene glycol alginate ester (PGA) and beet-derived pectin (BD-pectin), OSA starch. These may be used alone or in combination.
Incidentally, the biopolymer can be used together with other nonionic or negatively charged surfactants. It is desired that the surfactant is usually used so as to be contained in the mineral additive of the present invention in the range of from 0 to 20% by weight.

The amount of the biopolymer to be used may be generally about 0.01 to 10 wt.%, preferably 0.1 to 5 wt.%, and preferably around 1% wt. with respect to the total amount of non-dried product containing colloidal particles, but these ranges do not restrict the scope of the invention because they may vary depending on differences in the type of biopolymer and concentration of colloidal particles. The weight ratio of biopolymer to iron-containing colloidal particles is generally at least about 1:10,000 or higher (e.g. more biopolymer in comparison to colloidal particle mass).

The combination step (iii) preferably comprises dispersing the complex, in the form of particles, in the supporting medium.

The method is particularly suitable for manufacturing the composition of the invention when the composition comprises a supporting medium as described hereinbefore.

While the above summarizes the present invention, it will become apparent to those skilled in the art that modifications, variations and alterations may be made without deviating from the scope and spirit of the present invention as described and claimed herein. The invention will now be further illustrated in the following, non-limiting examples.

### Example 1

Colloidal particles *of Hoodia gordonii* extract were prepared. 2.4 g of Hoodia extract (73% steroidal glycosides) was dissolved in 22.4 g ethanol at room temperature. 4.8 g of stabiliser polyvinylpyrrolidone (PVP) was dissolved in the same ethanolic solution. The the resulting mixture was rapidly introduced into a beaker containing 170 g demineralised water at room temperature. The water phase was agitated using a Silverson mixer set at 5,500 rpm. The resulting suspension of colloidal particle was recovered by centrifugation and dispersed in pure water. Particle size determined using TEM and DLS was less than 400 nm.

### Example 2

Colloidal particles of *Hoodia gordonii* extract were prepared. 5 g of Hoodia extract (73% steroidal glycosides) was dissolve in 95 g ethanol at room temperature. 1 g of stabiliser Tween 80 was dissolved in the 199 g demineralised water. 5 g of the ethanolic solution of Hoodia extract was rapidly introduced in to a beaker containing the solution of Tween 80 at room temperature. During the mixing, the water phase was agitated using a Silverson mixer set at 5,500 rpm. The resulting suspension of colloidal particles comprising Hoodia gordonii extract can be further concentrated by evaporation of the ethanol. The colloidal particle are recovered by either centrifugation or complete (freeze or spray) drying of the suspension. Particle size determined using TEM and DLS was less than 500 nm.

### Example 3

Beverage contaning colloidal particles of *Hoodia gordonii* extract was prepared.
A concentrated suspension of colloidal particles recovered by centrifugation in Example 1 was carefully diluted with water to a 0.5 % total extract concentration. The resulting beverage had a liquid like appearance with slightly greenish colour. The beverage showed excellent stability, no sedimentation was observed for the six months period of storage at ambient temperature. The maximum particle size remained less than 350 nm.

While described in terms of the presently preferred embodiments, it is to be understood that such disclosure is not to be interpreted as limiting. Various modifications and alterations will no doubt occur to one skilled in the art after having read the above disclosure. Accordingly, it is intended that the appended claims be interpreted as covering all such modifications and alterations as falling within the true spirit and scope of the invention.

## Claims

1. A composition comprising colloidal particles of Hoodia plant extract, the extract comprising more than about 60%, of steroidal glycosides, by weight of the extract, wherein at least 90% of the particles have a particle size in the range of from about 10,000 nm to about 10 nm and the particles comprise at least about 2% of steroidal glycosides by weight of the particles.

2. A composition according to claim 1 wherein at least 90% by weight of the particles have a particle size of less than 5000 nm.

3. A composition according to claim 1 wherein at least 90% by weight of the particles have a particle size of less than 1000 nm.

4. A composition according to claim 1 wherein at least 90% by weight of the particles have a particle size of less than 500 nm.

5. A composition according to claim 1 wherein the particles are dispersed in a supporting medium.

6. A composition according to claim 1 wherein the plant is selected from *Hoodia gordonii*, *Hoodia currorii* subsp. *currorrii* and *Hoodia currorii* subsp. *lugardii.*

7. A composition according to claim 1, where in the plant is *Hoodia gordonii*.

8. A composition according to claim 1, wherein the steroidal glycosides having the formula: 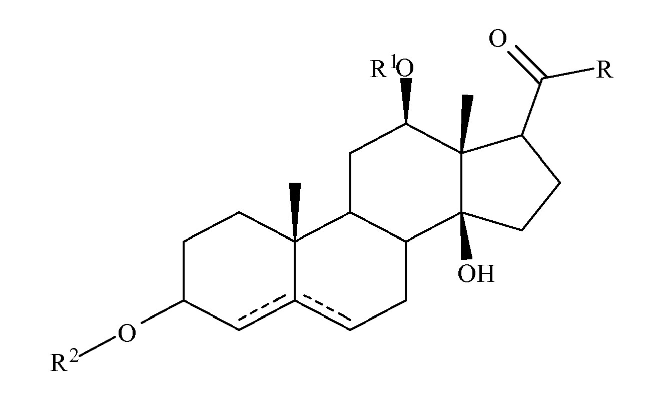 wherein R = alkyl;
R1 = H, alkyl, tiglyol, benzoyl or any other organic ester group;
R2 = H or one or more 6-deoxy carbohydrates, or one or more 2,6-dideoxy carbohydrates, or glucose molecules, or combinations thereof;
and wherein the broken lines indicate the optional presence of a further bond between carbon atoms C4 and C5 or between carbon atoms C5 and C6, or mixtures thereof.

9. A composition according to claim 1, wherein the steroidal glycoside has the formula:

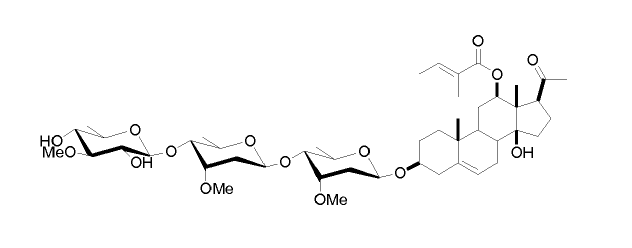

10. A composition according to claim 1 wherein the colloidal particles further comprise a water insoluble ingredient.

11. A composition according to claim 10 wherein the water insoluble ingredient is selected from the group consisting of oils, fats, waxes, their esters, vitamins, minerals, polymers and biopolymers.

12. An edible aqueous dispersion comprising the composition of claim 1.

13. A composition according to claim 1 which is selected from the group consisting of a food composition, a personal care composition, and a pharmaceutical composition.

14. A composition of claim 13 wherein the composition is food composition.

15. A composition according to claim 14 wherein the composition is a beverage, preferably a protein or tea-based beverage.

16. A composition comprising colloidal particles of Hoodia plant extract, wherein at least 90% of the particles have a particle size in the range of from about 10,000 nm to about 10 nm and the particles comprise at least about 60% of steroidal glycosides by weight of the particles.

17. A method of manufacturing a composition comprising of steroidal glycosides colloidal particles, the process comprising the steps of:
(i) preparing colloidal particles comprising steroidal glycoside;
(ii) recovering the particles; and
(iii) combining the colloidal particles with a supporting medium.

18. A method according to claim 16 wherein the colloidal particles are recovered in step (ii) as particles and wherein step (iii) comprises dispersing the particles in the supporting medium.

19. A method of managing body weight or in the dietary control of obesity the method comprising administering to a human the food composition of claim 13.
